# EUROPEAN PATENT APPLICATION

(11) **EP 3 815 615 A1**
(43) Date of publication of application: **05.05.2021**
(21) Application number: 19205820.4
(22) Date of filing: 29.10.2019
(51) Int. Cl.: A61B 8/08

(54) **SYSTEMS AND METHODS FOR POSITIONING ULTRASOUND PATCHES**

(71) Applicant: Koninklijke Philips N.V., 5656 AG Eindhoven (NL)
(72) Inventor: VERBAKEL, Frank, 5656 AE Eindhoven (NL); NOTTEN, Marc Godfriedus Marie, 5656 AE Eindhoven (NL); HAKKENS, Franciscus Johannes Gerardus, 5656 AE Eindhoven (NL); MERT, Aydin, 5656 AE Eindhoven (NL); RIJKEN, Antonius Maria, 5656 AE Eindhoven (NL); GROEN, Johanneke Gerrigje, 5656 AE Eindhoven (NL)
(74) Representative: Philips Intellectual Property & Standards

(57) **Abstract**

The invention provides for a method and an apparatus (300) for positioning an ultrasound probe, in particular an ultrasound patch, on a surface of a subject. The apparatus includes first fixing unit (210) and second fixing unit (310) adapted to be fixed to a surface of the subject at a first location and second location, respectively. The apparatus further comprises a holding unit (260) adapted to receive the ultrasound probe. The holding unit is adapted to be coupled with the first and second fixing units at the surface of the subject. When the holding unit is coupled to the two fixing units, the position of the holding unit can be adjusted relative to the two fixing units , e.g. to to fine-tune the captured image or to compensate for errors in placement or subject movement without requiring a total repositioning of the probe and holding unit.

## Description

### FIELD OF THE INVENTION

This invention relates to the field of ultrasound imaging, and in particular the field of ultrasound imaging patches.

### BACKGROUND OF THE INVENTION

Typically, ultrasound devices are probes operated by ultrasound experts to capture the required images. The probes are very useful for diagnostics, but less so for monitoring and visualization during procedures such as vascular access, CTO crossing, AV Fistula and below the knee (BTK) treatments. The main reason for this is that the imaging must be performed in a hands free manner to allow the clinician to perform the procedure.

Many different ultrasound patches have been designed for this purpose, however whilst they solve problem of hands-free imaging, a variety of issues still remain present. For example, current ultrasound patches provide no means of: correcting small misalignments of the patch in placement and movements of the subject during treatment/monitoring; creating stable images over long monitoring periods; and handling changes in focus depth for imaging vessels at different depths.

During fixation of an ultrasound probe to the surface of a subject, small movements are possible. In addition, during treatment the skin will move with respect to the vessel due to manipulation of the skin while inserting devices and movement of the subject. Both will result in a less accurate final image and may even require replacement of the patch.

Further, the vessels that need to be imaged often vary in depth under the skin. Some vessels are immediately under the skin, while in other areas the vessel are up to 50mm, or more, below the surface of the skin. This requires different focus depths, which is not currently possible with a single standard probe. Currently clinician are sometimes using gloves filled with US gel for this purpose, however the use is quite cumbersome.

There is therefore a need for a more reliable means of positioning and ultrasound probe, and in particular an ultrasound patch, at the surface of a subject.

### SUMMARY OF THE INVENTION

The invention is defined by the claims.

According to examples in accordance with an aspect of the invention, there is provided an apparatus for positioning an ultrasound probe on a surface of a subject, the apparatus comprising:
a first fixing unit, wherein the first fixing unit is adapted to be fixed to a surface of the subject at a first location;
a second fixing unit, wherein the second fixing unit is adapted to be fixed to a surface of the subject at a second location different from the first location;
a holding unit, wherein the holding unit is adapted to receive the ultrasound probe; and
wherein, the holding unit is adapted to be coupled with the first fixing unit and the second fixing unit at the surface of the subject and wherein, when the holding unit is coupled to the first fixing unit and the second fixing unit, the position of the holding unit is adjustable relative to the first fixing unit and the second fixing unit.

The apparatus provides for a means of hands-free ultrasound imaging where the position of the ultrasound transducer may be adjusted, by way of manual adjustment by a user or by way of automatic adjustment using a mechanical adjustment mechanism, to fine-tune the captured image after the ultrasound image has been initially fixed to the subject.

In this way, the position of the ultrasound probe may be adjusted to compensate for errors in placement or subject movement without requiring a total repositioning of the probe and holding unit.

In an embodiment, the position of the holding unit is adjustable by way of one or more of:
a translational adjustment; and
a rotational adjustment.

In this way, the position of the probe may be adjusted in any way required. The position of the probe may be adjusted in a plane tangential to the surface of the subject, or in a plane tangential to the surface of the subject.

In an embodiment, the apparatus further comprises a permanent fixation unit adapted to fix the holding unit to the surface of the subject such that the position of the holding unit cannot be altered.

In this way, when the correct position is found, the holding unit (and so the ultrasound probe) may be fixed in place for long term imaging.

In an embodiment, the apparatus comprises a temporary handle adapted to be removably coupled to the holding unit.

In this way, a handle may be provided to improve the accuracy of placement and adjustment without requiring the handle to remain a part of the apparatus when worn by the subject.

In a further embodiment, the temporary handle is coupled to the holding unit by way of a magnet.

In an embodiment, the holding unit comprises a stand-off portion adapted to adjust a distance between the ultrasound transducer and the surface of the subject.

In this way, different focus depths may be imaged by the transducer.

In a further embodiment, the stand-off portion has an aperture window adjacent the surface of the subject, and wherein, if the stand-off portion has a thickness below a given value, the aperture window comprises a first portion and second portions located at either side of the first portion.

In a further embodiment, the stand-off portion comprises a stand-off material adapted to provide an acoustic coupling between the ultrasound transducer and the surface of the subject. In this way, the acoustic coupling may be improved.

In a further embodiment, the stand-off material comprises an aqua-gel material.

In this way, the acoustic coupling may be maintained for long imaging periods.

In an embodiment, the first fixing unit and the second fixing unit comprise:
an adhesive patch adapted to conform and adhere to the surface of the subject; and
a receiving unit comprising a recessed portion adapted to receive the holding unit.

In a further embodiment, the holding unit comprises a protruding portion adapted to be received by the receiving unit.

According to examples in accordance with an aspect of the invention, there is provided a method for positioning an ultrasound probe on a surface of a subject, the method comprising:
fixing a first fixing unit to a surface of the subject at a first location;
fixing a second fixing unit to a surface of the subject at a second location different from the first location;
coupling a holding unit, adapted to hold an ultrasound transducer, to the first fixing unit and the second fixing unit at the surface of the subject; and
adjusting the position of the holding unit about the first fixing unit and/or the second fixing unit.

In an embodiment, adjusting the position of the holding unit comprises one or more of:
performing a translational adjustment; and
performing a rotational adjustment.

In an embodiment, the holding unit comprises a stand-off portion and wherein adjusting the position of the holding unit further comprises providing a stand-off material to the stand-off portion.

In an embodiment, the method further comprising fixing the holding unit to the surface of the subject after the adjustment of the position of the holding unit.

According to examples in accordance with an aspect of the invention, there is provided an ultrasound patch comprising:
a first array group comprising a primary transducer array having a first orientation; and
a second array group comprising at least two secondary transducer arrays each having an orientation different from the first orientation,
wherein the second array group is arranged such that the at least two secondary transducer arrays are located at either side of the primary transducer array.

The ultrasound patch provides for a means of capturing multiple image streams, each of which may be adapted for a different purpose. In other words, the ultrasound patch may provide for a means of multifunction image capture within a single unit, meaning that the ultrasound patch does not need to be swapped on a function by function basis. Thus, the ultrasound patch provides for a more efficient workflow.

In an embodiment, the at least two secondary transducer arrays have the same orientation.

In a further embodiment, the at least two secondary transducer arrays are perpendicular to the primary transducer array.

In this way, it is possible to capture ultrasound data in two planes located either side of a primary perpendicular plane, thereby providing a cross sectional view at either side of the primary imaging plane.

In an embodiment, the at least two secondary transducer arrays have different orientations.

In an embodiment, the first array group comprises a plurality of primary transducer arrays.

In this way, it is possible to capture multiple views in the first orientation without the need for repositioning the ultrasound patch.

In an embodiment, the first array group is adapted to capture preliminary ultrasound data for positioning the ultrasound patch.

In an embodiment, the second array group is adapted to capture preliminary ultrasound data for positioning the ultrasound patch.

In this way, the position of the holding unit may be adjusted based on incoming ultrasound data without requiring all of the array groups to be activated at once.

In an embodiment, the primary transducer array and the at least two secondary transducer arrays comprise CMUTs.

In an embodiment, the ultrasound probe comprises a flip chip connection.

In this way, the elements of the ultrasound transducer array may be directly bonded to a PCB or flexible circuit of the ultrasound probe, thereby reducing the amount of space between transducer elements and improving the coverage of the transducer array.

According to examples in accordance with an aspect of the invention, there is provided a system for positioning an ultrasound probe at the surface of a subject, the system comprising:
the ultrasound patch as described above; and
an apparatus (100) for positioning an ultrasound patch (110) on a surface of a subject, the apparatus comprising:
   a first fixing unit (120), wherein the first fixing unit is adapted to be fixed to a surface of the subject at a first location;
   a second fixing unit (130), wherein the second fixing unit is adapted to be fixed to a surface of the subject at a second location different from the first location;
   a holding unit (140), wherein the holding unit is adapted to receive the ultrasound patch; and
   wherein, the holding unit is adapted to be coupled with the first fixing unit and the second fixing unit at the surface of the subject and wherein, when the holding unit is coupled to the first fixing unit and the second fixing unit, the position of the holding unit is adjustable relative to the first fixing unit and the second fixing unit.

The apparatus provides for a means of hands-free ultrasound imaging where the position of the ultrasound patch may be adjusted to fine-tune the captured image after the ultrasound image has been initially fixed to the subject.

In this way, the position of the ultrasound patch may be adjusted to compensate for errors in placement or subject movement without requiring a total repositioning of the probe and holding unit.

In an embodiment, the apparatus further comprises a stand-off portion to adjust a distance between the ultrasound patch and the surface of the subject.

In this way, the position of the focus depth of the apparatus may be changed without requiring the ultrasound patch to be changed. It should be noted that the actual focus depth of the probe does not change, rather the position of the focal point within the subject changes using the stand-off portion.

In an embodiment, the stand-off portion has an aperture window adjacent the surface of the subject, and wherein, if the stand-off portion has a thickness below a given value, the aperture window comprises a first portion aligned with primary transducer array and second portions aligned with the at least two secondary transducer arrays.

In this way, the aperture window may be aligned with the transducer arrays in order to allow ultrasound waves to reach the subject, but without reducing the stability of the stand-off portion.

According to examples in accordance with an aspect of the invention, there is provided a method for positioning an ultrasound patch on a surface of a subject, the method comprising:
providing the ultrasound patch to a holding unit, wherein the ultrasound patch comprises:
   a first array group comprising a primary transducer array having a first orientation; and
   a second array group comprising at least two secondary transducer arrays each having an orientation different from the first orientation,
wherein the second array group is arranged such that the at least two secondary transducer arrays are located at either side of the primary transducer array.
fixing a first fixing unit to a surface of the subject at a first location;
fixing a second fixing unit to a surface of the subject at a second location different from the first location;
coupling the holding unit to the first fixing unit and the second fixing unit at the surface of the subject; and
adjusting the position of the holding unit about the first fixing unit and the second fixing unit.

In an embodiment, the method further comprises:
obtaining preliminary ultrasound data from the first array group; and
adjusting the position of the holding unit about the first fixing unit and the second fixing unit based on the preliminary ultrasound data.

In an embodiment, the method further comprises:
obtaining preliminary ultrasound data from the second array group; and
adjusting the position of the holding unit about the first fixing unit and the second fixing unit based on the preliminary ultrasound data

In this way, the position of the holding unit may be adjusted based on incoming ultrasound data without requiring all of the array groups to be activated at once.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an ultrasound diagnostic imaging system to explain the general operation;
Figure 2A shows an apparatus for positioning an ultrasound probe on a surface of a subject;
Figure 2B shows the apparatus of Figure 2A, wherein the holding unit is coupled to the first fixing unit and the second fixing unit;
Figure 3 shows an example implementation 200 of the apparatus of Figures 2A and 2B;
Figure 4 shows an example of the apparatus of Figure 3 when applied to the arm of a subject;
Figure 5A shows a cross section view of the apparatus when applied to the surface of the subject;
Figure 5B shows the apparatus of Figure 5A with a stand-off portion;
Figure 6 shows an example of the apparatus comprising a temporary handle;
Figure 7 shows a method of the invention;
Figure 8 shows an example of an ultrasound patch;
Figure 9 shows the ultrasound patch of Figure 9 when housed within the apparatus of Figure 5B;
Figure 10 shows a further example of an ultrasound patch; and
Figure 11 shows several further examples of ultrasound patches.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides for an apparatus for positioning an ultrasound probe on a surface of a subject. The system includes first fixing unit and second fixing unit adapted to be fixed to a surface of the subject at a first location and second location, respectively. The system further comprises a holding unit adapted to receive the ultrasound probe.

The holding unit is adapted to be coupled with the first and second fixing units at the surface of the subject and, when the holding unit is coupled to the first fixing unit and the second fixing unit, the position of the holding unit is adjustable relative to the first fixing unit and the second fixing unit.

A further aspect of the invention provides an ultrasound patch comprising a first array group comprising a primary transducer array having a first orientation and a second array group comprising at least two secondary transducer arrays each having an orientation different from the first orientation. The second array group is arranged such that the at least two secondary transducer arrays are located at either side of the primary transducer array.

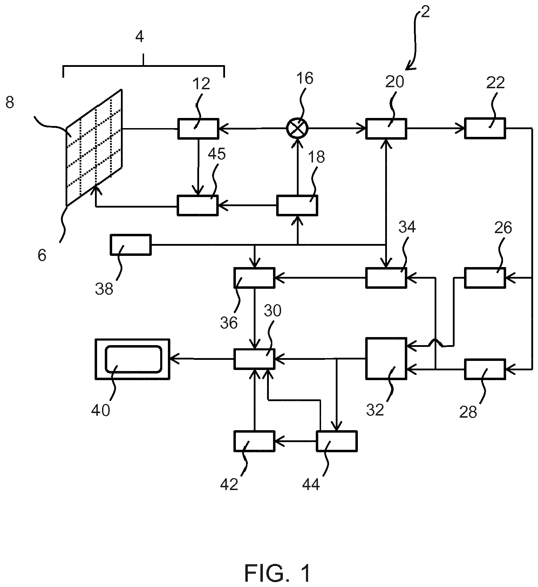

The general operation of an exemplary ultrasound system 2 will first be described, with reference to Figure 1.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

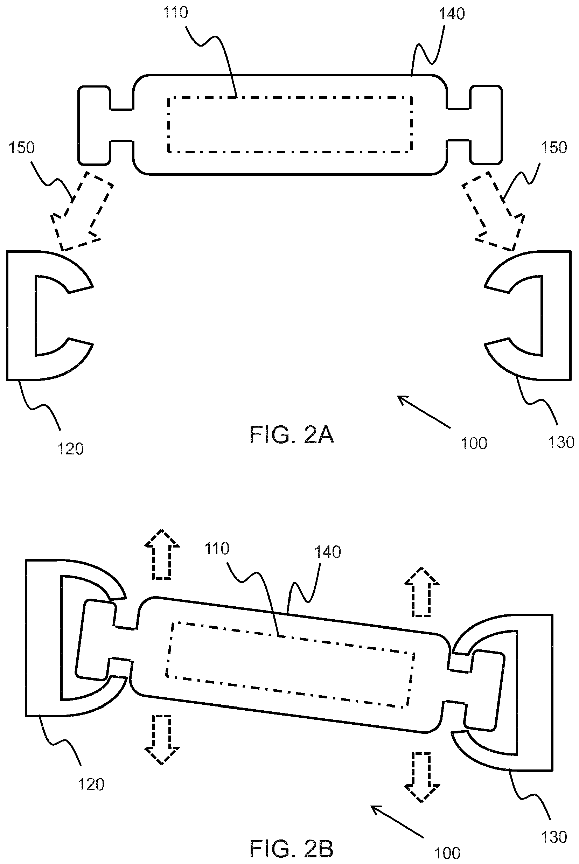

Figure 2A shows an apparatus 100 for positioning an ultrasound probe 110 on a surface of a subject. This probe ultrasound probe 110 can be arranged to operate with the ultrasound system 2 in a similar manner as described above in relation to the array transducer probe 4.

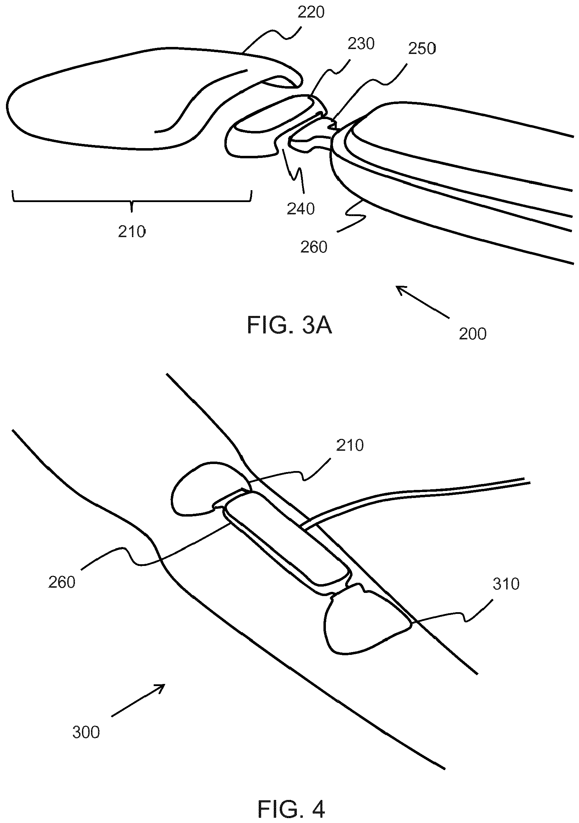

The apparatus comprises a first fixing unit 120 and a second fixing unit 130, which are adapted to be fixed to a surface of the subject at a first location and a second location, respectively. The first fixing unit and the second fixing unit may be fixed to the surface of the subject bay any suitable means. Examples of some such suitable means are discussed further below with reference to Figure 3.

The apparatus further comprises a holding unit 140 adapted to receive the ultrasound probe. Put another way, the holding unit holds the ultrasound in place during an ultrasound scan. It should be noted that the ultrasound probe 110 may be integrally formed with the holding unit.

The holding unit is adapted to be coupled 150 with the first fixing unit and the second fixing unit at the surface of the subject.

Figure 2B shows the apparatus of Figure 2A, wherein the holding unit 140 is coupled to the first fixing unit 120 and the second fixing unit 130.

When the holding unit is coupled to the first fixing unit and the second fixing unit, the position of the holding unit is adjustable relative to the first fixing unit and the second fixing unit as indicated by the arrows.

The position of the holding unit may be adjustable by way a translational adjustment or a rotational adjustment depending on the implementation of the holding unit and the first fixing unit and the second fixing unit.

The apparatus provides a means for fine tuning the location of the ultrasound probe after the initial placement of the probe to generate the best possible image, with minimal obtrusion for the clinician. In other words, the position of the ultrasound probe may be slightly adjusted after the holding unit has been coupled to the first fixing unit and the second fixing unit, which secure the holding unit next to the surface of the subject. Thus, the user is not required to perform both operations of holding the ultrasound transducer at the surface of the subject and fine tuning the position of the transducer.

Accordingly, the apparatus provides for an increase in the image quality and accuracy captured by the ultrasound transducer.

The coupling between the holding unit and the first fixing unit and second fixing unit may provide, for example, fine tuning of the position of the holding unit of 3 - 5mm after placement of the holding unit at the surface of the subject. The fine tuning of the position of the holding unit may be greater than 5mm according to the application of the ultrasound probe. For example, the holding unit may receive an ultrasound transducer at the arm of a subject and hold it at the surface of the skin, whilst the apparatus still provides for some movement of 3 - 5 mm after coupling.

By manipulating only one side of the holding unit, such as the left or right side, a rotational adjustment may be performed. In combination with a translational adjustment, the apparatus provides for adjustment to correct any given misplacement or movement of the skin with respect to the vessels/vessels being imaged.

Further, the apparatus may be easily adjusted during ongoing measurements or monitoring to re-optimize the image in a fast and simple manner. After an adjustment, a user may continue to use the ultrasound system in a hands-free manner.

The apparatus combines a reusable ultrasound module, the ultrasound transducer, and a disposable holding unit with a fine tuning mechanism. The components of the apparatus may be modular, with each unit being adapted depending on the given application. Using such a modular approach, the apparatus may be adjusted to meet the requirements of a specific application, whereas the ultrasound module can be made universally applicable to the holding unit. In other words, the holding unit may be provided in a number of forms adapted to fit different situations, whilst still being able to receive the same ultrasound module.

Figure 3 shows an example implementation 200 of the apparatus of Figures 2A and 2B.

The example shown in Figure 3 includes an exploded view of the first fixing unit 210, which includes an adhesive patch 220 adapted to conform and adhere to the surface of the subject and a receiving unit 230 comprising a recessed portion 240 adapted to receive a protruding portion 250 of the holding unit 260. The receiving unit may be rigid or flexible. It shall be understood by the skilled in the art person that all illustrated holding units herein (such as 140, 260) may be adapted to have a protruding portion arranged to be placed into the receiving unit.

Figure 4 shows an example 300 of the apparatus of Figure 3, with the second fixing unit 310 shown, when applied to the arm of a subject 320.

The apparatus may further include a permanent fixation unit adapted to fix the holding unit to the surface of the subject such that the position of the holding unit cannot be altered.

The permanent fixation unit may be any means of attaching the holding unit to the surface of the subject such that the holding unit does not move relative to the surface of the subject. The fixing unit may attach the holding unit to the surface of the subject on a semi-permanent basis, such as by way of micro-porous tape or a plaster, or on a permanent basis, such as by way of an adhesive requiring solvent to remove.

For example, for subjects undergoing long term monitoring, it might be required to have a better fixation then obtained by a sticking plaster. After initial placement and fine tuning of the location of the holding unit, a second sticking plaster may be applied to firmly fixate the ultrasound patch to the skin. In this case the apparatus may remain on the skin for multiple days. Only a part of the ultrasound patch may be covered by the sticking plaster or the full ultrasound patch can be covered by a sticking plaster to fully seal the area.

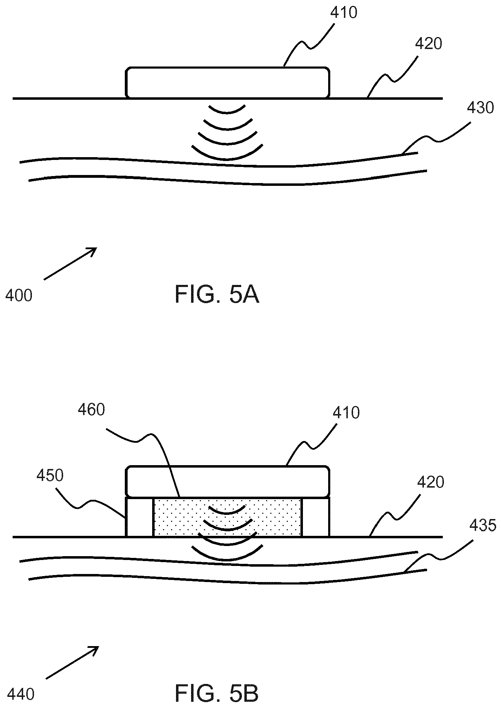

Figure 5A shows a cross section view 400 of the apparatus 410 when applied to the surface 420 of the subject. In the example shown in Figure 5A, the apparatus is being employed to image a vessel 430 that is located at a depth between 10 - 80mm, or more, below the surface of the skin.

Figure 5B shows a cross section view 440 of the apparatus 410 when applied to the surface 420 of the subject. In the example shown in Figure 5B, the apparatus is being employed to image a vessel 435 that is located at a depth less 10 mm below the surface of the skin.

Figure 5B further shows the apparatus comprising a stand-off portion 450 adapted to adjust a distance between the ultrasound transducer and the surface of the subject.

The ultrasound probe may include an ultrasound lens for focussing the beam of ultrasound signals at a single point, for example in applications where electronic ultrasound beam steering or focussing is not possible, such as for 1D arrays of transducers used for 2D imaging.

The stand-off portion provides a means for altering the location of the focus depth of the ultrasound probe, which is typically located at 10 to 45 mm from the ultrasound probe depending on the lens of the probe itself, when held at the surface of the subject for imaging regions of interest close to the surface, which would otherwise not be in focus. In other words, the stand-off portion and stand-off material may be used to compensate for a fixed focus point of an ultrasound probe and lens combination and provides for a means of changing the perceived focus in the body of the subject.

Put another way, the apparatus may be combined with various stand-off portions in order to generate various focus depths depending on the location of the holding unit without the need to change the ultrasound image generation device. Using dedicated stand-off portions with heights varying between 0 mm and 15 mm, it is possible to have the current focus depth of the ultrasound device vary according to the current imaging application. It should be noted that a stand-off portion of any height may be used, for example, a stand-off portion with a height of more than 15mm may be used.

For example, when imaging a shallow vessel, such as the vessel 430 shown in Figure 5A, the holding unit may be directly applied to the surface 420 of the subject to place the focus of the ultrasound waves at the vessel depth 430. A stand-off material, such as an acoustic gel may be placed between the holding unit and the surface of the subject.

As a further example, when imaging a vessel at the skin level, such as the vessel 435 shown in Figure 5B, the apparatus may comprises a stand-off portion 450 with a thickness of up to 15mm to focus the ultrasound waves at the skin level. The stand-off portion may include a stand-off material 460, such as an acoustic coupling material. The stand-off material may be any material capable of providing an acoustic coupling between the ultrasound probe within the holding unit and the surface of the subject.

In addition to altering the depth of the focus point of the ultrasound probe, the stand-off portion and stand-off material may be used to perform a translational adjustment for fine tuning the height of the ultrasound probe, i.e. the distance between the ultrasound probe and the surface of the subject. Further, the stand-off portion and stand-off material may be employed to perform a rotational adjustment of the ultrasound probe with respect to the surface of the subject.

In some cases, long term monitoring is required. During an extended period of monitoring time, some liquid based stand-off material may dry out, leading to areas where imaging is distorted. Accordingly, an aqua-gel material may be provided as the stand-off material within stand-off portion in order to reduce the drying effect over time, thereby providing for accurate image for longer time periods, such as up to several days.

Put another way, the stand-off portion and stand-off material may provide for an offset in height of the ultrasound probe without disturbing the ultrasound signal. Various commercial available materials may be used as stand-off material. In an example, the stand-off material may be standard ultrasound gel held in a suitable container. The stand-off material may also permit for movement of the apparatus after initial application. In other words, the stand-off material may aid the movement of the holding unit during the fine tuning adjustments described above.

The stand-off portion provides an additional aspect to the modular nature of the apparatus, which may employ a single reusable module containing all of the electrical components, such as the ultrasound transducers, and several disposable holding units and stand-off portions for varying focus depths (0-15mm) all with the built in fine tuning mechanism as described above.

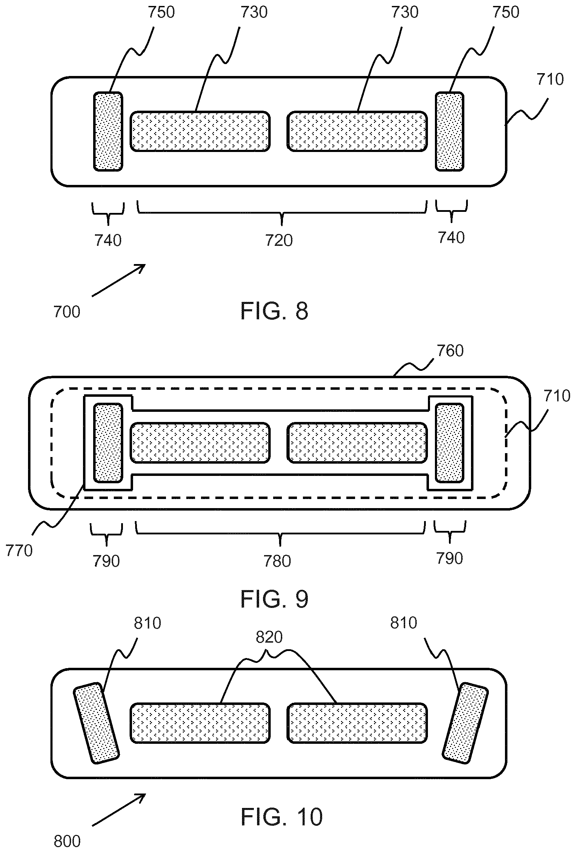

The stand-off portion is further elaborated below with reference to Figure 9.

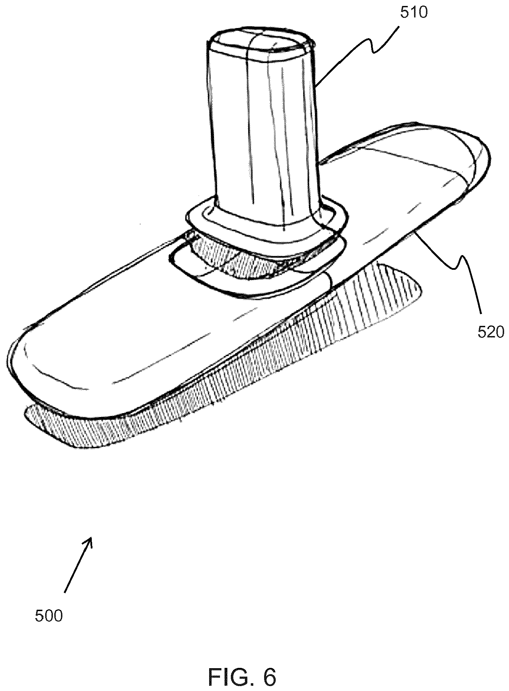

Figure 6 shows an example of the apparatus 500 comprising a temporary handle 510 adapted to be removably coupled to the holding unit 520. The temporary coupling of the temporary handle to the holding unit may be achieved by way of a magnet located on the temporary handle and a corresponding magnet located on the holding unit.

The temporary handle may be used to facilitate the placement of the holding unit on the skin of a subject. In this way, the initial placement of the holding unit may be more accurate, but also the accuracy of the fine tuning adjustments may be increased.

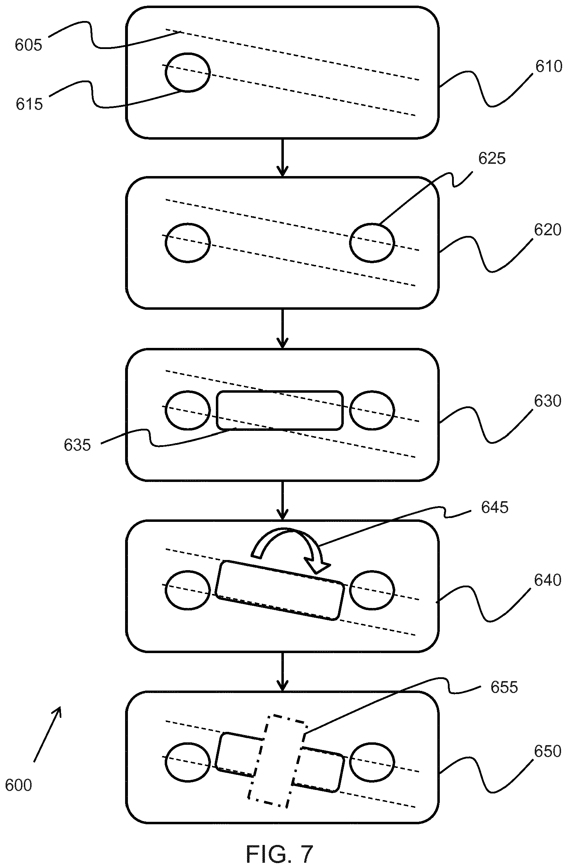

Figure 7 shows a method 600 for positioning an ultrasound probe on a surface of a subject. In the example shown in Figure 6, the ultrasound probe is being positioned on a surface proximate to a region of interest to be imaged 605 such as vessel.

In step 610, a first fixing unit 615 is fixed to the surface of the subject at a first location.

In step 620, a second fixing unit 625 is fixed to the surface of the subject at a second location different from the first location.

In step 630, a holding unit 635, adapted to hold an ultrasound transducer, is coupled to the first fixing unit and the second fixing unit at the surface of the subject.

In step 640, the position of the holding unit undergoes an adjustment 645 about the first fixing unit and/or the second fixing unit in order to bring the holding unit in line with the region of interest such as vessel to be imaged. The fixing units' arrangement of this invention provides the user with a flexibility, common to diagnostic ultrasound probes, of moving the transducer around for the optimized image quality acquisition.

In step 650, the holding unit is fixed to the surface of the subject after the adjustment of the position of the holding unit by way of a permanent fixing unit 655. This step allows the user to fix the position of the transducer in relation to the region of interests, once an optimal position has been identified.

Alternatively, the first and second fixing units may be first affixed to the holding unit and ultrasound probe, and then placed on the surface of the subject. The ultrasound probe may then be employed to check the positon of the holding unit is largely correct before fixing the first and second fixing units to the surface of the subject. The position of the holding unit may then be fine-tuned as described above.

Figure 8 shows an example 700 of an ultrasound patch 710 comprising a first array group 720 comprising a primary transducer array 730 having a first orientation and a second array group 740 comprising at least two secondary transducer arrays 750 each having an orientation different from the first orientation.

As shown in Figure 8, the second array group 740 is arranged such that the at least two secondary transducer arrays 750 are located at either side of the primary transducer array 730. In this example, the first array group 720 comprises two primary transducer arrays.

The ultrasound patch 710 may be used as the ultrasound probe 110 used in the apparatuses described above.

The configuration of the ultrasound patch, such as the ultrasound patch 710 shown in Figure 8, depends on the clinical application of the patch and the user requirements. For example, the clinical requirements may include: a means of navigating the ultrasound patch to the region of interest; guiding the positioning of the device within the region of interest; monitoring the subject for parameters such as the diameter of a vessel and blood flow; guiding a procedure, such as positioning a catheter; monitoring the outcome of a procedure; and the like.

In the example shown in Figure 8, the at least two secondary transducer arrays 750 may be used to help position the ultrasound patch 710 over the region of interest. By providing a secondary transducer array at either side of the primary transducer array, in addition to providing appropriate clinical views, the accuracy of the positioning may be increased. Further, combining this feature with the fine tuning capabilities of the apparatus described above, the accuracy of the placement of the ultrasound transducer patch may be increased further.

It has been recognized that during an interventional procedure, when a catheter is placed within the vessel. The two secondary arrays 750 enable the user to track, whether the catheter enters the region of interest and exists it even if the primary ultrasound transducer 730 is not fully aligned with the vessel's axis, thereby providing only partial image of the anatomy.

For example, when using the ultrasound patch to monitoring a procedure on a blood vessels, both secondary transducer arrays may be used to generate a cross-sectional view of the vessel of interest and help to guide the placement of the first array group in plane with the vessel in order to acquire an accurate longitudinal image of the vessel of interest. In addition, after placement has been completed, the secondary transducer arrays may be used to determine the blood flow and diameter of the vessel, thereby increasing the functionality of the ultrasound patch.

The primary and secondary transducer arrays may utilize CMUT cells. CMUT cells enable optimal configurations to support monitoring applications as well as image guidance applications. In this way, multiple transducer arrays can be used for "real-time" imaging whilst still using single read-out electronics.

As a CMUTs are only active when a DC bias voltage is applied, the separate transducer arrays can be individually activated, while still sharing the beam forming channels of all of the transducer arrays. Therefore, only a single read-out system is needed for all of the transducer arrays. The switching of the DC bias voltage may be automated and at sufficient high speed, such that a real-time image of the region of interest can be acquired.

In the context of clinical applications, the primary ultrasound transducer 730 provides for a large field of view. For example, when the secondary transducer arrays have been utilized to guide the placement of the primary ultrasound transducer array on top of a vessel of interest, a full view of the issue, such as a stenosis, can be visualized.

The primary and secondary transducer arrays may perform both 2D and 3D ultrasound imaging depending on the application.

Figure 9 shows the ultrasound patch 710 of Figure 8 when held within an apparatus as described above, which comprises a stand-off portion 760 having an aperture window 770 adapted to be positioned adjacent the surface of the subject.

If the stand-off portion has a thickness below a given value, for example less than 5mm thick, such as less than 3mm, the aperture window comprises a first portion 780 aligned with primary transducer array and second portions 790 aligned with the at least two secondary transducer arrays.

By shaping the aperture window in this way, the stiffness of the stand-off portion and stand-off material can be increased, thereby ensuring good acoustic contact between the ultrasound patch and the surface of the subject. For example, a stiffener may be provided in the stand-off material at a non-imaging location so that the stiffener does not interfere with the imaging process. For larger thickness, no H-shape is allowed as material properties in this case prevent good contact with device and skin.

Figure 10 shows an example 800 of an ultrasound patch where the secondary transducer arrays 810 are oriented at different angles to each other and are not perpendicular to the primary transducer arrays 820.

The particular arrangement shown in Figure 10 is optimized for performing accurate blood flow measurements using the secondary arrays. In order to perform accurate blood flow measurements, there has to be motion in the direction of the ultrasound beam. If the flow is perpendicular to the beam, there is no relative motion from pulse to pulse. Accordingly, by aligning the primary transducer array along the length of a vessel, the secondary transducers are prevented from being perpendicular to the blood flow, thereby ensuring the accurate measurement of the flow. Therefore, the secondary transducers allow not only acquiring the cross sections of the vessel in an optimal manner but also enabling monitoring of the blood flow passing through the region of interest.

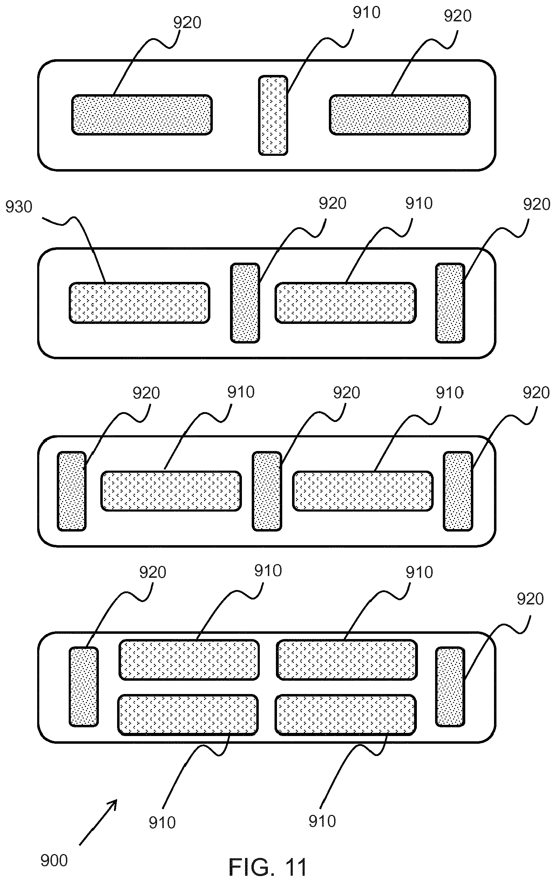

Figure 11 shows various examples 900 of ultrasound transducer patches utilizing different configurations of primary transducer arrays 910 and secondary transducer arrays 920. As can be seen from the examples, any number and arrangement of primary and secondary arrays may be utilized according to the requirements of the given application. Further, the ultrasound patched may include one or more tertiary transducer arrays 930 adapted to compliment the primary transducer arrays and secondary transducer arrays, but which do not fall within the first array group or the second array group.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. An apparatus (100) for positioning an ultrasound probe (110) on a surface of a subject, the apparatus comprising:
a first fixing unit (120), wherein the first fixing unit is adapted to be fixed to a surface of the subject at a first location;
a second fixing unit (130), wherein the second fixing unit is adapted to be fixed to a surface of the subject at a second location different from the first location;
a holding unit (140), wherein the holding unit is adapted to receive the ultrasound probe; and
wherein, the holding unit is adapted to be coupled with the first fixing unit and the second fixing unit at the surface of the subject and wherein, when the holding unit is coupled to the first fixing unit and the second fixing unit, the position of the holding unit is adjustable relative to the first fixing unit and the second fixing unit.

2. The apparatus (100) as claimed in claim 1, wherein the position of the holding unit (140) is adjustable by way of one or more of:
a translational adjustment; and
a rotational adjustment.

3. The apparatus (100) as claimed in any of claims 1 to 2, wherein the apparatus further comprises a permanent fixation unit (655) adapted to fix the holding unit to the surface of the subject such that the position of the holding unit cannot be altered.

4. The apparatus (100) as claimed in any of claims 1 to 3, wherein the apparatus comprises a temporary handle (510) adapted to be removably coupled to the holding unit.

5. The apparatus (100) as claimed in claim 4, wherein the temporary handle (510) is coupled to the holding unit by way of a magnet.

6. The apparatus (100) as claimed in any of claims 1 to 5, wherein the holding unit (140) comprises a stand-off portion (450) adapted to adjust a distance between the ultrasound transducer and the surface of the subject.

7. The apparatus (100) as claimed in claim 6, wherein the stand-off portion (450) has an aperture window (770) adjacent the surface of the subject, and wherein, if the stand-off portion has a thickness below a given value, the aperture window comprises a first portion and second portions located at either side of the first portion.

8. The apparatus (100) as claimed in any of claims 6 to 7, wherein the stand-off portion (450) comprises a stand-off material (460) adapted to provide an acoustic coupling between the ultrasound transducer and the surface of the subject.

9. The apparatus (100) as claimed in claim 8, wherein the stand-off material (460) comprises an aqua-gel material.

10. The apparatus (100) as claimed in any of claims 1 to 9, wherein the first fixing unit and the second fixing unit comprise:
an adhesive patch (220) adapted to conform and adhere to the surface of the subject; and
a receiving unit (230) comprising a recessed portion adapted to receive the holding unit.

11. The apparatus (100) as claimed in claim 10, wherein the holding unit comprises a protruding portion (250) adapted to be received by the receiving unit.

12. A method (600) for positioning an ultrasound probe on a surface of a subject, the method comprising:
fixing (610) a first fixing unit to a surface of the subject at a first location;
fixing (620) a second fixing unit to a surface of the subject at a second location different from the first location;
coupling (630) a holding unit, adapted to hold an ultrasound transducer, to the first fixing unit and the second fixing unit at the surface of the subject; and
adjusting (640) the position of the holding unit about the first fixing unit and the second fixing unit.

13. The method (600) as claimed in claim 12, wherein adjusting (640) the position of the holding unit comprises one or more of:
performing a translational adjustment; and
performing a rotational adjustment.

14. The method (600) as claimed in claim 13, wherein the holding unit comprises a stand-off portion and wherein adjusting the position of the holding unit further comprises providing a stand-off material to the stand-off portion.

15. The method (600) as claimed in any of claims 12 to 14, wherein the method further comprising fixing (650) the holding unit to the surface of the subject after the adjustment of the position of the holding unit.
